# EUROPEAN PATENT SPECIFICATION

(11) **EP 2 143 369 B1**
(45) Date of publication and mention of the grant of the patent: **27.06.2012**
(21) Application number: 09013351.3
(22) Date of filing: 04.03.2004
(51) Int. Cl.: H04B 7/06, H04B 7/08

(54) **Capsule-type medical system**
Medizinisches Kapselsystem
Système médical de type capsule

(43) Date of publication of application: 13.01.2010
(62) Divisional of application: 04717286.1
(73) Proprietor: Olympus Corporation, Shibuya-ku Tokyo 151-0072 (JP)
(72) Inventor: Fujita, Manabu, Hachioji-shi Tokyo 192-8512 (JP); Uchiyama, Akio, Hachioji-shi Tokyo 192-8512 (JP)
(74) Representative: von Hellfeld, Axel

(56) References cited:
- EP-A- 0 667 115
- EP-A2- 1 260 176
- JP-A- 2001 231 186
- US-A1- 2003 085 994

## Description

### Technical Field

The present invention relates to a capsular medical system which is inserted in the human body and obtains medical information on the body.

### Background Art

As an example of a conventional art of a capsular medical system which obtains information on the living body by a capsular in-body unit that is inserted in the body cavity and transmits by radio the obtained information to an extracorporeal device, Japanese Unexamined Patent Application Publication No. 2001-46357 discloses the capsular medical system in which the extracorporeal device comprises a plurality of receiving antennas and furthermore suggests a method for estimating the position of the capsular in-body unit as a transmitting source based on the receiving intensities of the receiving antennas.

In Japanese Unexamined Patent Application Publication No. 2001-46357, the position of the in-body unit is calculated by detecting the signal intensity from the in-body unit side by using a plurality of receiving antennas. Therefore, the signal levels of the signals received by the receiving antennas need to be compared on the extracorporeal device side and thus the circuit structure for detecting the position is complicated.

Further, there is a drawback that the signal from the in-body unit side is not efficiently received. That is, the plurality of receiving antennas are arranged and however the best antenna is not selected for reception. Therefore, there is a drawback that the signal from the in-body unit side is not efficiently received.

The present invention is devised in the above-mentioned points. It is one object of the present invention to provide a capsular medical system which monitors the communication state or receiving state of the in-body unit with the simple structure.

Further, it is another object of the present invention to provide a capsular medical system which monitors the communication state with the in-body unit with the simple structure and which is used for the estimation of the position of the in-body unit and for the selection of the best antenna based on the monitoring result.

Furthermore, it is another object of the present invention to provide a capsular medical system which selects the best antenna and sends medical information data.

US 2003/0085994 A1 relates to a capsule type medical device wherein signals are sent by radio waves from a single or multiple antennas provided in an external unit located outside of a living body to a capsule inserted into a living body to obtain living body information. The signals are received by the antenna on the capsule side and the radio wave strength data is obtained. Based on the radio wave strength data, living body information is sent from the capsule side to the external unit by using a transmission power suitable for the transmission. The radio wave strength data is sent to the external unit side, and the external unit side receives living body information by selecting and using an antenna, which can receive data with the highest radio wave strength.

### Disclosure of Invention

According to the present invention, a capsular medical system comprises the features of the independent claim.

Preferred embodiments of the capsular medical system are defined by the dependent claims.

### Brief Description of the Drawings

The first to fourth and sixth embodiment do not form part of the invention but are useful for understanding the invention.
Figs. 1 to 3C relate to the first exemplary embodiment useful for understanding the present invention, Fig. 1 is a diagram showing the entire structure of a capsular medical system according to the first exemplary embodiment useful for understanding the present invention;
Fig. 2A is a diagram showing the structure of an electric system of an in-body unit;
Fig. 2B is a diagram showing the data structure of medical data transmitted from the in-body unit;
Fig. 3A is a block diagram showing the structure of an extracorporeal device;
Fig. 3B is a flowchart schematically showing the operation details for calculating a data transfer speed;
Fig. 3C is a flowchart schematically showing the operation details for calculating the data transfer speed and the position estimation;
Fig. 4 is a block diagram showing the structure of an extracorporeal device according to the second exemplary embodiment useful for understanding the present invention;
Fig. 5 is a block diagram showing the structure of an extracorporeal device according to the third exemplary embodiment useful for understanding the present invention;
Figs. 6A to 6C relate to the fourth exemplary embodiment useful for understanding the present invention, Fig. 6A is an explanatory diagram of the operation according to the fourth exemplary embodiment useful for understanding the present invention;
Fig. 6B is a diagram showing an antenna stored in a memory and a data transfer speed;
Fig. 6C is a diagram for estimating the distance ratio based on a ratio value of reciprocal numbers of three data transfer speeds;
Figs. 7 to 12 relate to the fifth embodiment of the present invention, Fig. 7 is a diagram showing the entire structure of a capsular medical system according to the fifth embodiment of the present invention;
Fig. 8 is a circuit diagram showing the structure of an antenna unit;
Fig. 9 is a diagram showing an arrangement example of the antenna;
Fig. 10 is a flowchart showing the processing details in switching antennas;
Fig. 11 is a flowchart showing the processing details in the re-connecting operation upon disconnection shown in Fig. 10;
Fig. 12 is an explanatory diagram of the specific operation for switching the antennas during examining the body by using an in-body unit; and
Fig. 13 is a flowchart showing the processing details in switching antennas according to the exemplary sixth embodiment useful for understanding the present invention.

### Best Mode for Carrying Out the Invention

Hereinbelow, embodiments of the present invention and exemplary embodiments useful for understanding the present invention will be described with reference to the drawings.

### (First exemplary embodiment)

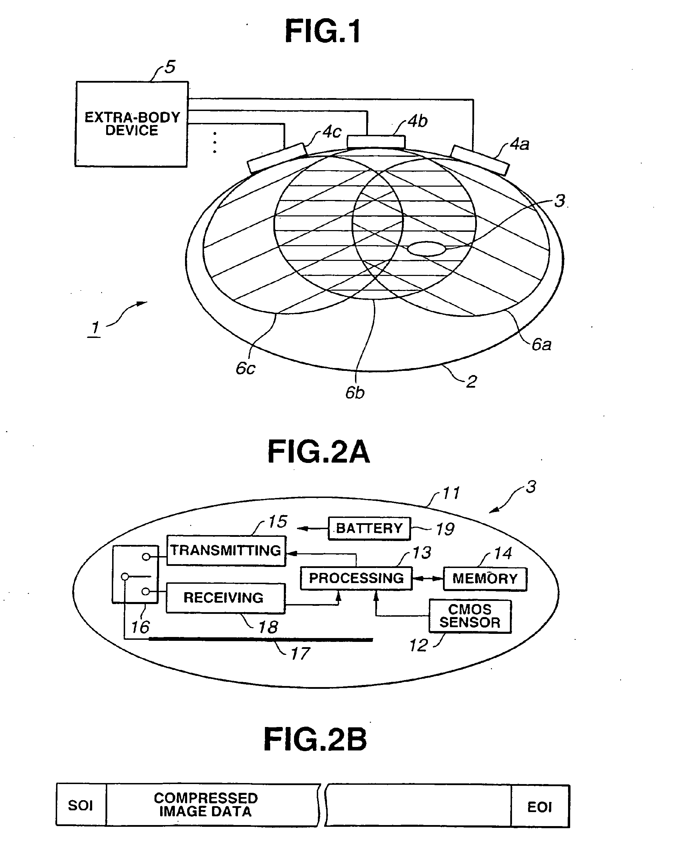

A first exemplary embodiment useful for understanding the present invention will be described with reference to Figs. 1 to 3C.

Referring to Fig. 1, a capsular medical system 1 comprises: a capsular in-body unit 3 inserted in a body 2; and an extracorporeal device 5 which is arranged outside the body 2 and receives medical data transmitted from the in-body unit 3. The extracorporeal device 5 receives the medical data transmitted from the in-body unit 3 through antennas 4a, 4b, ... arranged to the body surface of the body 2.

Fig. 1 schematically shows receiving and transmitting areas 6a, 6b, ... by elliptical lines. The receiving and transmitting are made through the antennas 4a, 4b, ....

Fig. 2A shows the structure of an electric system of the in-body unit 3. The in-body unit 3 comprises: a CMOS sensor 12 as an image pick-up element in a capsular sealed-container 11, which picks up the image; a processing circuit 13 which performs the signal processing of an image signal picked up by the CMOS sensor 12; and a memory 14 which temporarily stores or the like the image data processed by the processing circuit 13.

The in-body unit 3 accommodates: a transmitting circuit 15 which modulates with a radio frequency (RF) the image data stored in the memory 14 to obtain a transmitting signal; an antenna 17 which transmits the transmitting signal as electric waves to the extracorporeal device 5 via a receiving and transmitting switch 16; a receiving circuit 18 which demodulates the signal transmitted from the extracorporeal device 5 by the antenna 17; a battery 19 for supplying power necessary for the operation to the CMOS sensor 12 and the processing circuit 13 etc.; and the like.

As mentioned above, the processing circuit 13 forms compressed image data which is obtained by compressing the image picked up by the CMOS sensor 12. Upon transmitting the compressed image data, referring to Fig. 2B, an SOI symbol and an EOI symbol indicating the start and the end are added to the start and the end of the compressed image data and then are transmitted. That is, the SOI symbol and the EOI symbol are added to the start and the end of the medical data in units and then are transmitted.

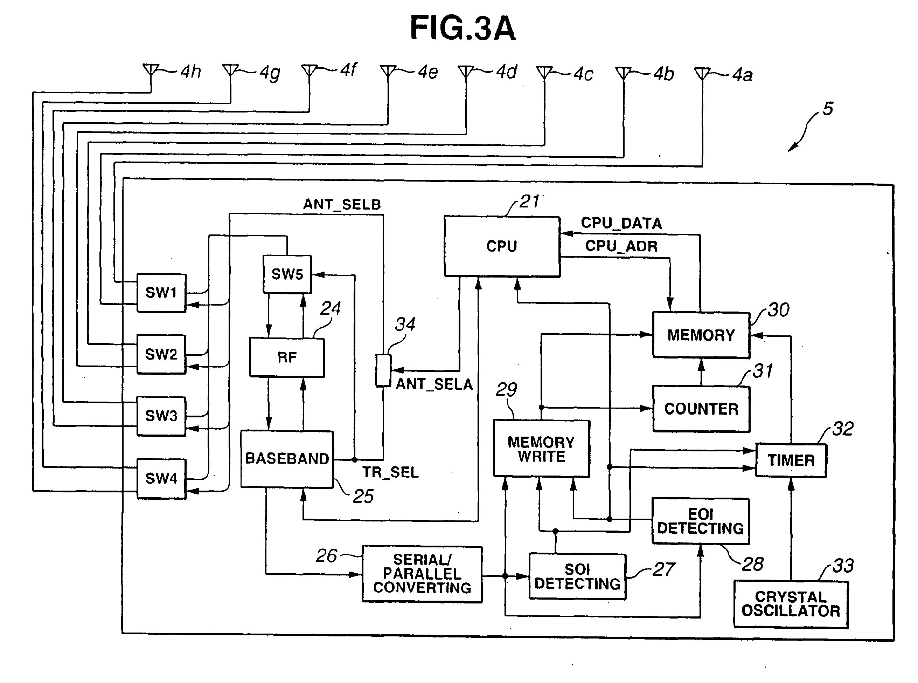

Fig. 3A shows the structure of the extracorporeal device 5. Referring to Fig. 3A, the extracorporeal device 5 comprises: a CPU 21 which controls the components of the extracorporeal device 5; switches SW1 to SW4 which switch the antennas 4a to 4h; a switch SW5 which switches the reception and transmission of the antennas 4a to 4h; an RF circuit 24 which demodulates a high-frequency signal received through the switches SW1 to SW5; and a baseband circuit 25 which performs the processing and the like for converting the signal demodulated by the RF circuit 24 into a baseband signal.

The extracorporeal device 5 further comprises: a serial/parallel converting circuit 26 which converts serial data into parallel data based on the baseband signal; an SOI detecting circuit 27 and an EOI detecting circuit 28 which detect the SOI symbol and the EOI symbol based on the parallel signal; a memory write circuit 29 which receives the parallel data, performs the memory write operation in response to an instruction for the start of image writing from the SOI detecting circuit 27, and stops the memory write operation in response to an instruction for ending to write the image from the EOI detecting circuit 28; and a memory 30 which stores the parallel data by the memory write circuit 29.

The extracorporeal device 5 comprises: a counter circuit 31 which counts the number of times for writing the parallel data to the memory 30 by the memory write circuit 29; a timer circuit 32 which counts the time from the timing for instructing the start of writing the image from the SOI detecting circuit 27 to the timing for instructing the end of writing the image by the EOI detecting circuit 28 (outputted in association with the EOI detection); a crystal oscillator 33 which supplies a reference clock of the timer operation to the timer circuit 32; and an antenna switching circuit 34 which is switched by signals from the CPU 21 and the baseband circuit 25 and switches the antenna selected upon receiving and transmitting the antennas 4a to 4h.

According to the first exemplary embodiment, the SOI symbol and the EOI symbol added to the start and end of the image data shown in Fig. 2B are detected and the time interval therebetween is counted by the timer circuit 32. Thereby, the amount of data of the image data transmitted to the extracorporeal device 5 from the in-body unit 3 is calculated and it is divided by the transmitting time which is counted by the timer circuit 32, thus to calculate the data transfer speed.

The CPU 21 transmits a command and the like to the in-body unit 3 side via the baseband circuit 25.

The baseband circuit 25 determines the receiving and transmitting state under the control of the CPU 21, controls the switching of the switch SW5 by a transmission switching signal (TR_SEL in Fig. 3A), and switches the reception and transmission.

The baseband circuit 25 controls the switching timing of an antenna switching signal (ANT_SELA in Fig. 3A) from the CPU 21, and can select a different signal for selecting the switches SW1 and SW4 (ANT_SELB in Fig. 3A) upon transmission or upon reception (that is, can switch the antenna to another antenna).

The data stored in the memory 30 is read by an address (CPU_ADR in Fig. 3A) from the CPU 21 and the data (CPU_DATA in Fig. 3A) is captured in the CPU 21.

As mentioned above, according to the first exemplary embodiment, the medical data in units is transmitted to the extracorporeal device 5 from the in-body unit 3 side. The extracorporeal device 5 switches the antenna and receives the medical data. In this case, the time from the SOI symbol to the EOI symbol as the start and end of the medical data is counted. The received medical data size in units is divided by the obtained time, thereby obtaining the data transfer speed upon using each of the antennas.

In this case, the SOI symbol and the EOI symbol are inevitably used for the normal transmission of the medical data. The time from the SOI symbol to the EOI symbol is counted without monitoring the signal level. Thus, it is possible to monitor the transmitting state of the medical data, that is, the receiving state of the transmitting information from the in-body unit 3.

Hereinbelow, a description is given of the operation of the capsular medical system 1 with the above structure.

The capsular in-body unit 3 is swallowed and then the image is picked up by the in-body unit 3. The extracorporeal device 5 receives the image data transmitted by radio.

In this case, the image picked up by the CMOS sensor 12 in the capsular in-body unit 3 becomes one sheet of the image data often being subjected to the compression processing. The SOI symbol indicating the start of the image data is added to the head of the image data corresponding to one sheet and the EOI symbol indicating the end of the image data are added to the end portion thereof, and the transmitting circuit 15 transmits the resultant data to the extracorporeal device 5 via the antenna 17.

In the extracorporeal device 5, the signal is received from the selected antenna 4i and is demodulated by the RF circuit 24. Further, the baseband circuit 25 restores the demodulated data into the data in the format of the compressed image data, and outputs the resultant data as the serial data.

The serial data outputted from the baseband circuit 25 is converted into the parallel data by the serial/parallel converting circuit 26, and is outputted to the SOI detecting circuit 27, the EOI detecting circuit 28, and the memory write circuit 29.

The SOI detecting circuit 27 inquires a pattern of the SOI symbol. Upon detecting the SOI symbol, the SOI detecting circuit 27 supplies the instruction for starting the image writing to the memory write circuit 29.

The memory write circuit 29 writes, to the memory 30, the parallel data received in response to the instruction for starting the image writing.

The counter circuit 31 counts the number of writing times for writing the data to the memory 30 from the memory write circuit 29, and stores the counted data to the memory 30.

The instruction for starting the image writing is inputted to the timer circuit 32. The timer circuit 32 counts the time from the timing for starting the image writing to the detection of the EOI symbol, and stores the counted time to the memory 30.

The EOI detecting circuit 28 detects the EOI symbol and then outputs the instruction for ending the image writing to the memory write circuit 29 and the timer circuit 32.

The memory write circuit 29 receives the instruction for ending the image writing and the stops the output to the memory 30.

The instruction for ending the image writing is also inputted to the CPU 21 and the CPU 21 reads from the memory 30 the image data size written by the counter circuit 31 and the time required for receiving the data, that is, the time from the detection of SOI symbol until the detection of EOI symbol written by the timer circuit 32.

Further, the CPU 21 divides the image data size by the time required for receiving of the data, that is, the time from the detection of SOI symbol until the detection of EOI symbol, and stores the data-transfer rate as the result (or data transfer speed) together with the selected antenna No. into the memory 30.

As mentioned above, the CPU 21 calculates the data-transfer rate every antenna and stores the calculated data-transfer rates of all the antennas 4a to 4h in the memory 30.

The CPU 21 switches the antenna to select an antenna which can increase the data-transfer rate to the highest level using the data-transfer rate stored in the memory 30, and after that, receives the image data from the in-body unit 3 by the selected antenna.

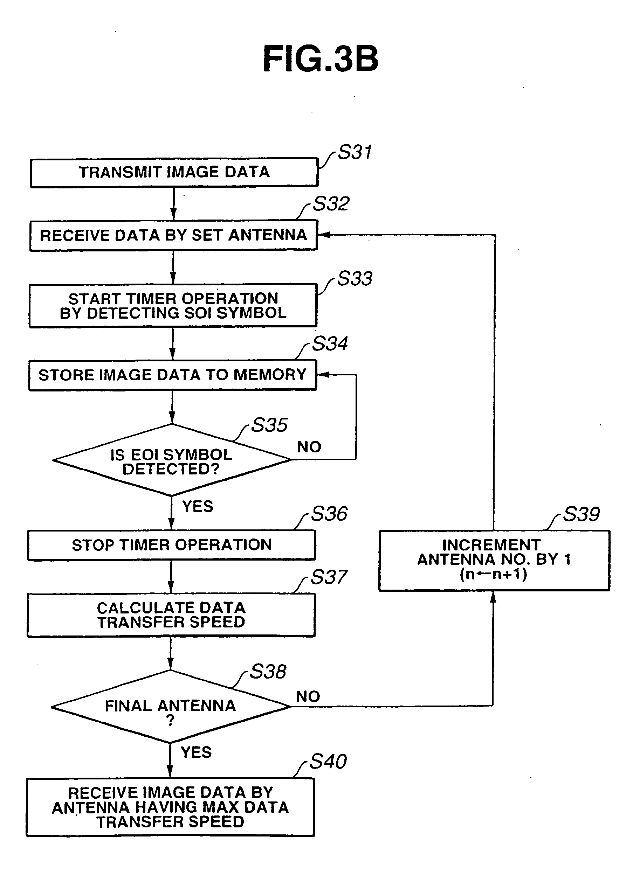

Fig. 3B schematically shows the operation for calculating the data-transfer rate according to the first exemplary embodiment.

In step S31, the in-body unit 3 adds the SOI symbol and the EOI symbol to the head and the end of the image data in units, respectively, and transmits the resultant data. In step S32, the image data is received by the antenna 4n (n = a) which is first set by the extracorporeal device 5.

In step S33, the extracorporeal device 5 starts the timer operation of the timer circuit 32 upon detecting the SOI symbol.

In step S34, the received image data is stored in the memory 30. As mentioned above, the image data transmitted from the in-body unit 3 is sequentially stored in the memory 30.

In step S35, the CPU 21 in the extracorporeal device 5 monitors whether or not the EOI symbol is detected. When the EOI symbol is not detected, the CPU 21 returns to step S34 whereupon the image data is continuously recorded.

In the meantime, upon detecting the EOI symbol, in step S36, the CPU 21 stops the timer operation of the timer circuit 32.

In step S37, the CPU 21 divides the data amount of the received image data by the counted-time of the timer circuit 32, and calculates the data transfer speed (data-transfer rate).

In step S38, the CPU 21 determines whether or not the antenna No. is the final No. of the antenna, i.e., 4j. If NO in step S38, in step S39, the CPU 21 increments the antenna No. n by 1 and returns to step S32 whereupon the CPU 21 performs the similar operation for the next antenna No., namely, the antenna 4b.

As mentioned above, the antenna No. is sequentially switched and the data transfer speed is calculated. Upon ending the processing for calculating the data transfer speed by the antenna 4j as the final antenna No., in step S40, the CPU 21 sets an antenna to that of the antenna No. for obtaining the maximum data transfer speed, and controls the operation for continuous receiving operation.

The three-dimensional position of the in-body unit 3 can be estimated based on the data-transfer rates obtained by the plurality of antennas. It is because the data-transfer rate decreases with the increase in distance.

That is, the data in units is transmitted by electric waves from the in-body unit 3 and the extracorporeal device 5 receives the electric waves via the antenna. Then, as the distance (between the in-body unit 3 and the antenna) increases from one distance range, the data-transfer rate is reduced.

As mentioned above, when the distance is some degree or more, the non-completion (no-ending) of the reception of the data as one unit is caused with the increase in distance. As a result, the request for transmitting the data is frequently outputted again in accordance with the increase in distance. Therefore, the distance between the in-body unit 3 and the antenna can be estimated based on the transfer rate of the data in units.

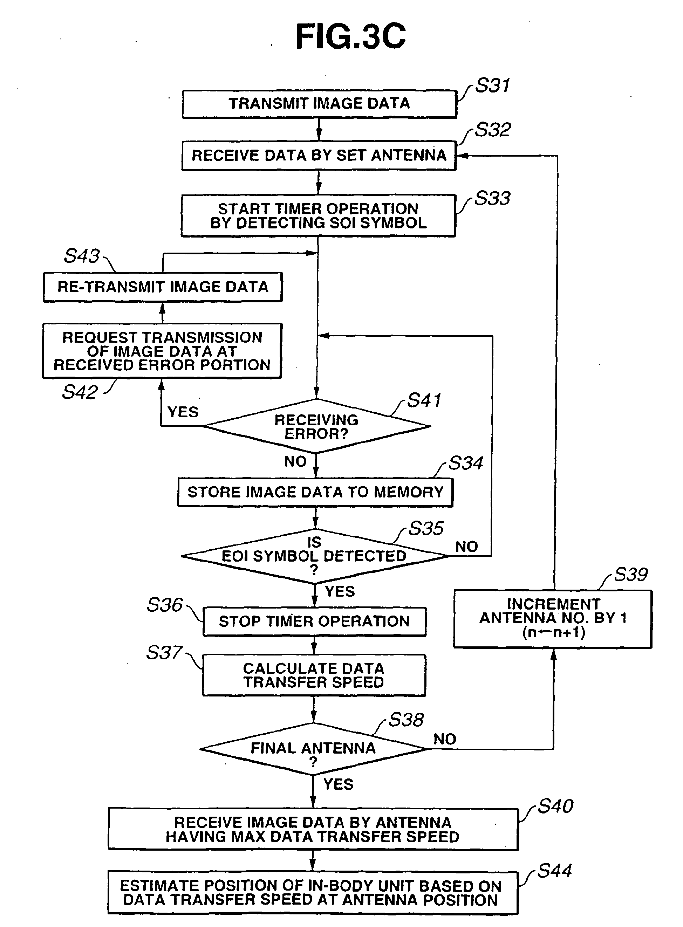

Fig. 3C schematically shows the operation details in this case. Referring to Fig. 3C, after processing in step S33 in Fig. 3C, in step S41, the CPU 21 in the extracorporeal device 5 determines whether or not a receiving error is caused. When the receiving error is received.and demodulated, the CPU 21 determines whether or not the error which cannot be corrected occurs.

When it is determined that the receiving error does not occur, the CPU 21 advances to step S34. When it is determined that the receiving error occurs, in step S42, the CPU 21 controls the operation for transmitting, to the in-body unit 3, a signal for requesting the transmission so as to transmit the image data just before the receiving error occurs. The in-body unit 3 receives the signal, in step S43, it transmits the image data again, and it returns to step S41. The extracorporeal device 5 receives the image data again and determines whether or not the receiving error occurs. When it is determined in step S35 that the EOI symbol is not detected, the processing routine returns to step S41.

Referring to Fig. 3C, after step S40 in Fig. 3B, the CPU 21 estimates the position of the in-body unit 3 based on the respective estimation of the distance from the data of the data-transfer rate at the antenna positions.

According to the first exemplary embodiment, it is possible to monitor the receiving state of signals from the in-body unit 3 with the simple structure and to select a best antenna from the monitoring result and receive the medical data.

That is, according to the first exemplary embodiment, a system for monitoring the receiving state by the signal level is not necessary and the receiving state is monitored without adding any specific hardware.

Further, it is possible to estimate the position of the in-body unit 3 by using the result of a tendency to reduce the data-transfer rate with the increase in distance.

### (Second exemplary embodiment)

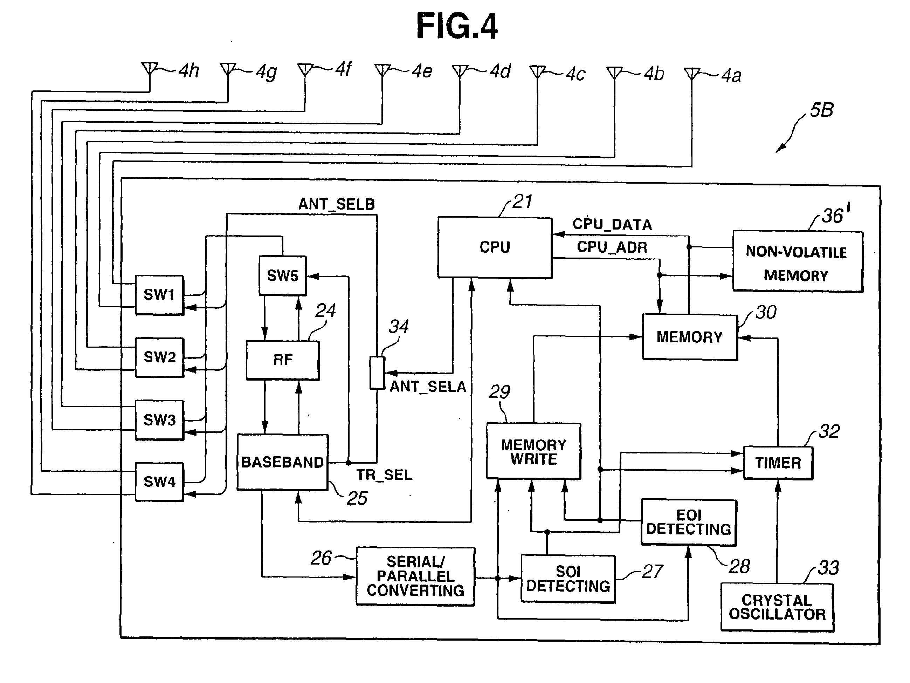

Fig. 4 shows the structure of an extracorporeal device 5B according to a second exemplary embodiment useful for understanding the present invention. According to the second exemplary embodiment, the size of the transmitted image data is constant.

Referring to Fig. 4, the extracorporeal device 5B is formed by adding a non-volatile memory 36 instead of the counter circuit 31 in the extracorporeal device 5 in Fig. 3A so as to write the size of the image data as one unit transmitted from the in-body unit 3 to the non-volatile memory 36.

Similarly to the first exemplary embodiment, the time required for transferring the image data is counted, the image data with the predetermined size written to the non-volatile memory 36 is divided by the time required for transferring the data, and the transfer speed is calculated.

Other structure and operation are the same as those according to the first exemplary embodiment. According to the second exemplary embodiment, the transfer speed can simply be calculated. Further, the best antenna is selected and the position is easily estimated.

### (Third exemplary embodiment)

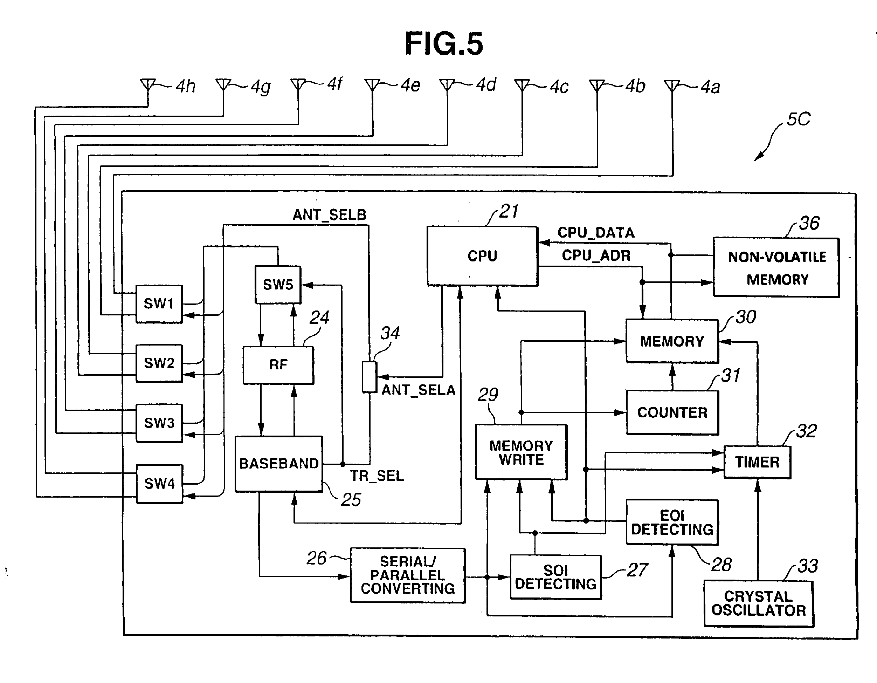

Next, a third exemplary embodiment useful for understanding the present invention will be described with reference to Fig. 5. Fig. 5 shows the structure of an extracorporeal device 5C according to the third exemplary embodiment useful for understanding the present invention.

The extracorporeal device 5C is formed by adding a non-volatile memory 36' to the extracorporeal device 5 shown in Fig. 3A for previously storing an allowable value of the transfer speed to the non-volatile memory 36'. Further, the CPU 21 compares the calculated transfer speed with the lowest allowable value of the transfer speed stored in the non-volatile memory 36'. When the calculated transfer speed is lower than the lowest allowable value of the transfer speed, the CPU 21 changes the selected antenna.

Other structure and operation are the same as those according to the first exemplary embodiment. According to the third exemplary embodiment, it is possible to prevent a long time selection of the antenna which is not suitable to the reception in the extracorporeal device 5C.

Therefore, the image data is efficiently received from the in-body unit 3. Other structure and operation have the same advantages as those according to the first exemplary embodiment.

### (Fourth exemplary embodiment)

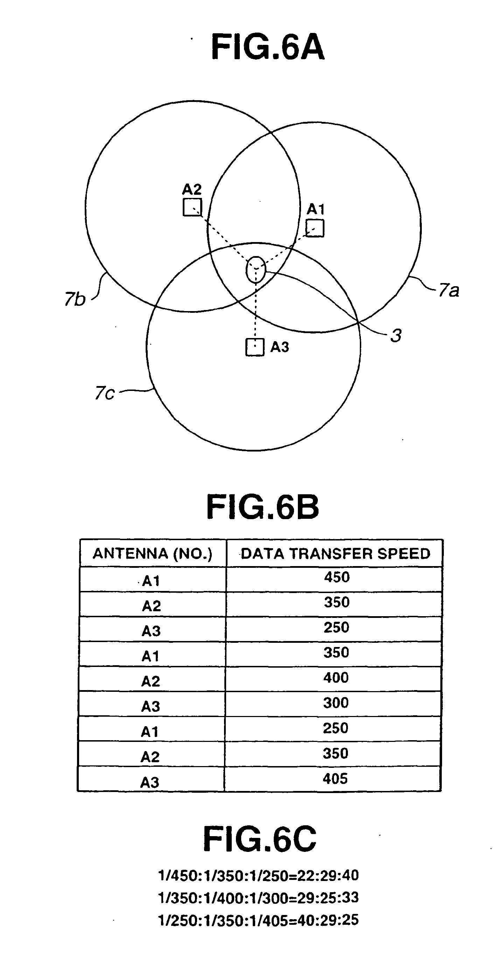

Next, a fourth exemplary embodiment useful for understanding the present invention will be described with reference to Figs. 6A to 6C. The fourth exemplary embodiment has the same structure as that according to the second exemplary embodiment.

Similarly to the second exemplary embodiment, the transfer speed is calculated. According to the fourth exemplary embodiment, the position of the in-body unit 3 is calculated by using the antenna which can communicate data at the allowable transfer speed or more, which will be described later.

First, the antennas which can communicate the data at the allowable transfer speed or more are designated by reference symbols A1, A2, and A3 shown in Fig. 6A for the purpose of a brief description.

That is, the three antennas A1 to A3 are within ranges 7a, 7b, and 7c in which the communication is possible from the in-body unit 3 at the allowable transfer speed or more.

In this case, referring to Fig. 6B, the memory 30 or non-volatile memory 36 stores the antenna (Nos.) A1 to A3 and the data transfer speed in each case.

Referring to Fig. 6B, the antennas A1 to A3 are sequentially switched cyclically and the image corresponding to one sheet is transmitted. By setting the processing as one cycle, the inverse ratio of the antenna transfer speeds is calculated respectively to set the ratio of distances, and the positions are estimated as shown in Fig. 6C.

In this case, performed is only the position estimation according to the distance ratio. However, the actual position is further estimated by previously measuring and obtaining the antenna transfer speed in the well-known position state.

As mentioned above, according to the fourth exemplary embodiment, it is possible to assure the state of transmitting the image data to the extracorporeal device 5 from the in-body unit 3 at the proper speed and to estimate the position of the in-body unit 3.

The three antennas A1 to A3 are switched cyclically as shown in Figs. 6A to 6C. However, the plurality of antennas which are approximate to the best state always receive the data by moving the in-body unit 3 and by switching the selected antenna. Further, the position of the in-body unit 3 is estimated (when the position is not estimated, the best or approximately best one antenna is selected. This case will be described according to the fifth embodiment later).

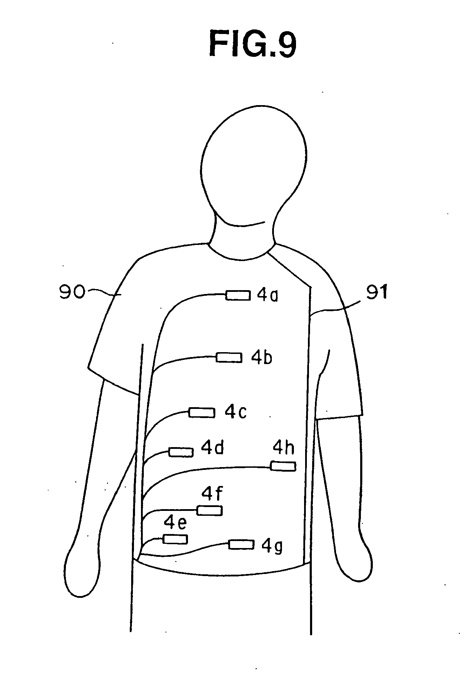

As will be described with reference to Fig. 9 later, the antennas' 4a, 4b, ... are swallowed in the in-body unit 3. When the in-body unit 3 is arranged along the moving route along the digestive tract, the in-body unit 3 can be moved and the position of the in-body unit 3 can be estimated by switching three or more antennas.

When it is determined based on the position estimation that the in-body unit 3 is moved, one antenna which is the most apart from the in-body unit 3 is not selected. In place thereof, the new antenna which is closer to the in-body unit 3 is selected and the three or more antennas including the selected antenna always receive the data at the proper receiving speed and the position of the in-body unit 3 can be estimated.

According to the first to fourth exemplary embodiment, the timing such as that of switching the antenna may be applied to the following embodiments.

### (Fifth embodiment)

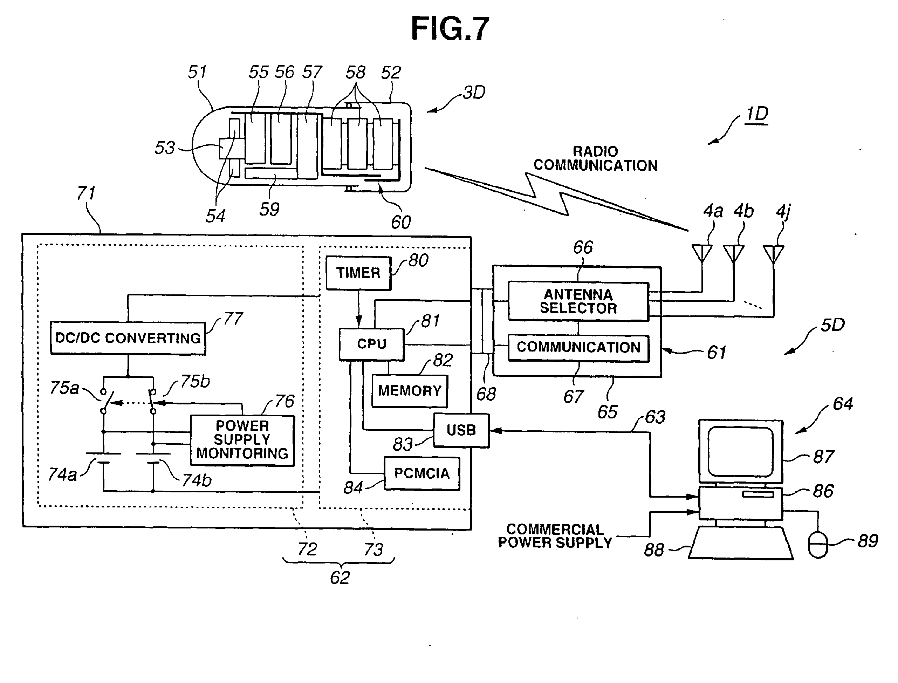

Next, a fifth embodiment of the present invention will be described with reference to Figs. 7 to 12. Fig. 7 shows the entire structure of a capsular medical system 1D according to the fifth embodiment.

The capsular medical system 1D comprises: a capsular in-body unit 3D which is inserted or swallowed in the body; and an extracorporeal device 5D which transmits data by radio to the in-body unit 3D.

The capsular medical system 3D comprises: an objective optical system 53 which is watertightly covered by a capsular accommodating container 51 and a back cover 52 which watertightly covers the back end side of the accommodating container 51 via O-ring, and which is disposed opposite the semi-spherical transparent portion; and white LEDs 54 as illuminating means at four positions on the perimeter of the objective optical system 53.

For example, a CMOS sensor 55 is arranged to the image forming position of the objective optical system 53. The CMOS sensor 55 includes, on the back side, a signal processing circuit 56 which performs the signal processing of the CMOS sensor 55, a communication circuit 57 which communicates data by radio, and a plurality of button-type batteries 58 which supply the power for operation to the CMOS sensor 55 and the signal processing circuit 56.

Further, the CMOS sensor 55 includes, at the adjacent side portion thereof, an antenna 59 which receives and transmits electric waves for radio communication with the extracorporeal device 5D and is connected to the communication circuit 57. Furthermore, the CMOS sensor 55 includes a switch 60 which switches on/off the power supply, adjacently to the batteries 58.

The extracorporeal device 5D comprises: an antenna unit 61 to which a plurality of antennas 4a to 4j are connected; a recording device 62, to which the antenna unit 61 is freely detachably connected and which records the image data; and an image display device 64 which is connected to the recording device 62 by a USB cable 63 and displays and edits the recorded image.

Further, the antenna unit 61 is included in a resin casing 65, and comprises: an antenna selector 66 which switches the antennas 4a to 4j connected respectively by coaxial cables; a communication circuit 67 which is connected to the antennas 4a to 4j via the antenna selector 66 and communicates data by radio using Bluetooth or the like; and a connector 68 for connection which is freely detachably connected to the recording device 62. The radio communication using the communication circuit 67 is not limited to the Bluetooth.

The recording device 62 is covered with a metal or plastic casing 71, and includes a power supply circuit block 72 and a processing circuit block 73. The power supply circuit block 72 comprises: batteries 74a and 74b arranged in parallel therewith; switches 75a and 75b directly connected to the batteries 74a and 74b; a power supply monitoring circuit 76 which monitors the voltages of the batteries 74a and 74b and switches on one of the switches 75a and 75b; and a DC/DC converter 77 which is connected to the on-battery and converts the power into DC power of a voltage value required by the processing circuit block 73.

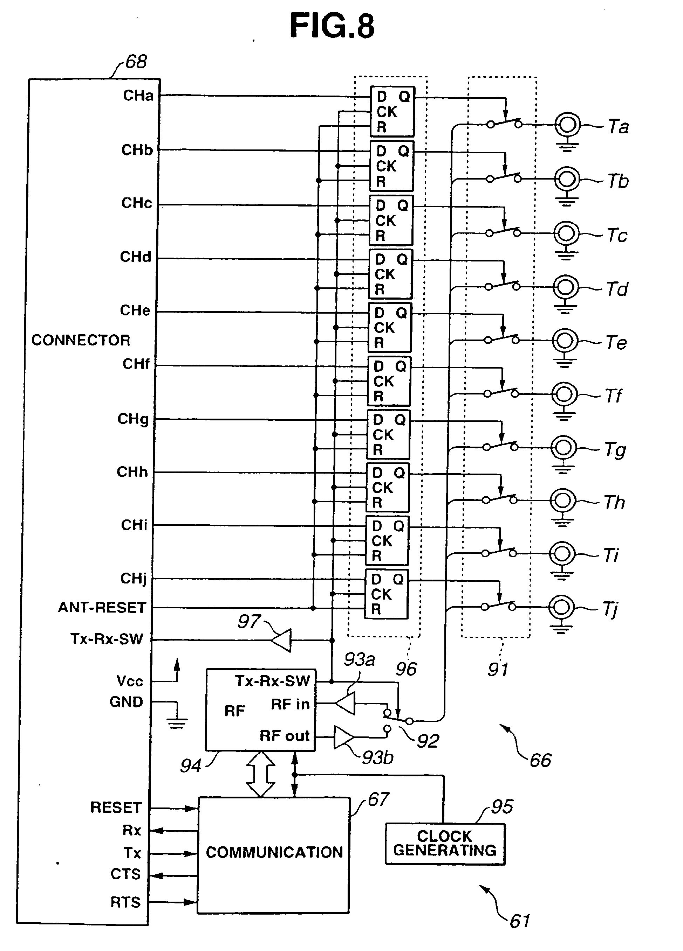

The DC power of the DC/DC converter 77 is supplied to the antenna unit 61 side via the connector 68 as well as the processing circuit block 73 (specifically, Vcc in Fig. 8).

The processing circuit block 73 comprises: a CPU 81 which controls the recording device 62 and the antenna unit 61; a memory 82 which is connected to the CPU 81 and is used for the data storage; a USB connector 83 which is connected to the CPU 81 and communicates data with the image display device 64; and a PCMCIA socket 84 which is connected to the CPU 81 and to which a memory card is freely detachably connected.

The CPU 81 is connected to a timer 80 and controls the operation for detecting the communication state at the time interval set by the timer 80. The operation for detecting the communication state causes the operation for selecting the antenna. That is, the operation for selecting the antenna is also performed at the time interval set by the timer 80.

Referring to Fig. 7, in addition to the entire control of the recording device 62, the single CPU 81 controls the communication with the antenna unit 61. Further, a CPU dedicated for communication control with the antenna unit 61 may be used.

The image display device 64 is, for example, a personal computer (abbreviated to a PC), and comprises: a main body 86 which performs the image display processing and the like; a monitor 87 which is connected to the main body 86 and displays the image; a keyboard 88 which is connected to the main body 86 and inputs a command, data or the like; and a mouse 89 which designates the position on the image and the like. Power for operation is supplied to the main body 86 from a commercial power supply via an insulating transfer (not shown).

Fig. 8 shows an example of the structure of the antenna unit 61.

Antenna connecting terminals Ta to Tj to which the antennas 4a to 4j are connected are connected respectively to an RF circuit 94 which receives and transmits an RF signal via an antenna selector 91, a reception and transmission switch 92, and buffers 93a and 93b. The RF circuit 94 is connected to a communication circuit 67 for communication operation.

A clock generating circuit 95 supplies reference clocks to the RF circuit 94 and the communication circuit 67 and then the RF circuit 94 and the communication circuit 67 perform the operation synchronized with the reference clocks. The communication circuit 67 communicates data with the CPU 81 in the processing circuit block 73 via the connector 68. Referring to Fig. 8, the reset operation and the communication of receiving data, transmitting data, transmitting permission, and transmitting request, and the like are performed via signals RESET, RX, TX, CTS, and RTS.

The RF circuit 94 detects the communication state with the CPU 81 in the communication circuit 67, generates a reception and transmission switching signal for switching the reception and the transmission (described as Tx-Rx-SW in Fig. 8), applies the reception and transmission switching signal to the reception and transmission switch 92, and selects one of the buffers 93a and 93b connected to the reception and transmission switch 92. Referring to Fig. 8, the communication state is set to the receiving state. In this state shown in Fig. 8, the buffer 93a is selected and the signal received by the antenna is inputted to a terminal RFin of the RF circuit 94.

On the other hand, in the transmitting state, the buffer 93b is selected and the RF signal outputted from a terminal RFout of the RF circuit 94 is outputted to the antenna side via the buffer 93b.

The CPU 81 controls the on/off operation of the antenna selector 91 via a latch circuit unit 96 connected via the connector 63, and selects and controls an antenna 4k (k = a to j) used for the reception and transmission.

Therefore, the CPU 81 connects channels CHa to CHj of the CPU 81 (via the connector 68) to a data input terminal D of a latch in the latch circuit unit 96, outputs data "H" as an antenna switching signal to the selector switch forming the antenna selector 91 from an output terminal Q of the latch, and sets the selector switch to which the antenna switching signal "H" is applied to the on-operation by a clock signal to a clock input terminal CK.

Fig. 8 shows an on-state of the selector switch connected to an antenna connecting terminal Ta. The selector switch is switched off by an antenna switching signal "L".

The reception and transmission switching signal for switching the reception and transmission switch 92 outputted from the RF circuit 94 is applied as the clock signal to the clock input terminal CK of each latch. Synchronously with the reception and transmission switching signal, each latch in the latch circuit unit 96 is operated. The reception and transmission switching signal is also transmitted to the CPU 81 from the connector 68 via a buffer 97. The CPU 81 grasps the timing for switching the antenna and the timing for switching the reception and transmission by the reception and transmission switching signal.

According to the fifth embodiment, the timing for switching the antenna is synchronously operated at the timing for switching the communication direction of the reception and transmission. In the case of switching to each antenna, the signals are transmitted and received and further the antenna is switched at the timing for switching the timing to that of the next transmission.

As mentioned above, the smooth transmission and reception are assured without switching the antenna during the transmission or reception and obstructing the communication (e.g., inhibition due to the communication abnormality and data lack).

The CPU 81 applies an antenna reset signal to a reset terminal R in all the latches of the latch circuit unit 96 via the connector 68, thereby resetting the operation.

For example, the CPU 81 outputs the antenna reset signal to reset all the latches in the initializing operation after turning on the power. After that, the CPU 81 sequentially outputs the data for switching and setting the antenna to the channels CHa to CHj via the connector 68.

Fig. 9 shows an example of the antennas 4a to 4j arranged to the body surface of the patient 2. The antennas 4i and 4j are arranged to the left and right on the rear surface side of the patient 2 (not shown). Further, antennas are arranged to have the sensitivity in the body upon using, as shown in the example of the arrangement of the antennas 4a to 4c which are obtained by cutting the body 2 into like rings shown in Fig. 1. The sensitivity outside the body is set to be low (by shielding) for preventing or reducing the outer disturbance.

A capsule signal is received at a wide area of the body by arranging a plurality of antennas.

The plurality of antennas are arranged to be contact to the body surface of the body 2 or adjacently thereto. Therefore, the antenna is enclosed with the thickness of the antenna of 2 mm or less and the size φ of 30 mm or less.

Referring to Fig. 9, the antennas 4a to 4j are sequentially arranged along the running direction of the digestive tract (according to the fifth embodiment, the total number of antennas is 10, however, it can be changed).

Further, in the example shown in Fig. 9, a vest-type antenna cable is pulled out around.

That is, according to a method for adhering the antennas to the body one by one, the operation is complicated. Therefore, the antennas 4a to 4j are sewed to a clothing 90 such as the vest and the clothing 90 is put on by the patient upon examination. Thus, the antenna is effectively set to the body.

The examination is performed by pulling out the cables in the case of the vest-type, and all the cables are concentrated to the front right of the body as shown in Fig. 9. The vest is opened and closed by a line 91 on the left.

In addition, the antenna may directly be attached to the body. The length necessary for pulling out the antenna cable is calculated and the length can be set to be proper.

As mentioned above, upon examination, a medical staff arranges the antennas 4a to 4j along the running direction of the digestive tract of the patient and switches the antennas 4a to 4j to change the antenna which is actually used for the communication. Thus, the antenna with the preferable communication state is selected.

According to the fifth embodiment, the in-body unit 3D communicates the data with the extracorporeal device 5D, and the in-body unit 3D detects the receiving strength of the signal received from the extracorporeal device 5D. Further, the in-body unit 3D transmits the detected receiving strength to the extracorporeal device 5D. Consequently, the extracorporeal device 5D obtains the receiving strength in the case of the antenna used for the communication and switches to the antenna with the high receiving strength so as to use the antenna to communicate with the in-body unit 3D which will be described later.

Thus, even when the in-body unit 3D moves along the digestive tract, the proper antenna is sequentially switched for selection and use. The image data picked-up by the in-body unit 3D is accurately obtained.

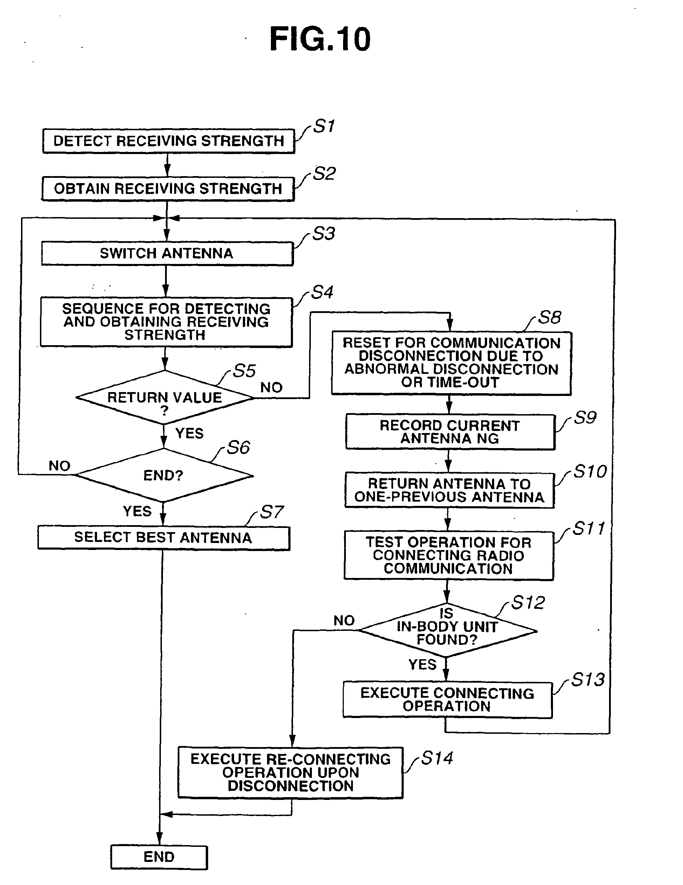

Next, the operation for switching (changing) the antenna will be described with reference to Fig. 10.

In step S1, the CPU 81 in the recording device 62 in the extracorporeal device 5D transmits a request for detecting the receiving strength to the in-body unit 3D via the antenna unit 61 after the time set by the timer 80 passes.

As mentioned above, according to the fifth embodiment, the communication state is detected by the time interval set by the timer 80.

In this case, the extracorporeal device 5D enters the transmitting state and the CPU 81 instructs the request or detection of the receiving strength to the in-body unit 3D via the communication circuit 67 in the antenna unit 61, and then the reception and transmission switch 92 is switched such that (the extracorporeal device 5D side) is on the receiving side.

In step S2, the in-body unit 3D receives the request for detecting the receiving strength, detects the receiving strength in the current antenna state (on the extracorporeal device 5D side), and transmits information on the detected receiving strength to the extracorporeal device 5D. The CPU 81 in the extracorporeal device 5D obtains the data on the detection of the receiving strength transmitted from the in-body unit 3D, and records the obtained data to a recording medium such as the memory 82 or the like.

In step S3, the CPU 81 issues an instruction for requesting the transmission to the communication circuit 67 in the antenna unit 61, and outputs, to the latch circuit unit 96, the antenna switching signal by which the next antenna is connected to be selected. In addition to the switching of the antenna by the latch circuit unit 96, the extracorporeal device 5D side is set to the transmitting state.

In step S4, the sequence for detecting and obtaining the receiving strength is performed. That is, as shown in step S1, the request for detecting the receiving strength is transmitted to the in-body unit 3D, thus to execute the operation for detecting and obtaining the receiving strength returned from the in-body unit 3D.

When the operation in step S4 is executed, in step S5, the CPU 81 determines whether or not the information on the detection of the receiving strength returned from the in-body unit 3D, namely, a return value of the deceiving strength is obtained. When the return value is obtained, in step S6, it is determined whether or not the operation ends. When the operation for all the antennas 4a to 4j does not end, the processing sequence returns to step S3 whereupon the above-mentioned operation is performed for the next antenna.

When the operation for all the antennas 4a to 4j ends, in step S7, the antenna with the highest receiving strength is selected as the best antenna, the antenna is set to a state for receiving the image data from the in-body unit 3D, and the antenna changing operation ends. Since the in-body unit 3D moves along the running direction of the digestive tract, the antenna is switched to the best one and the proper communication state is maintained so as to always and stably receive the image data picked-up by the in-body unit 3D.

In step S5, when the return value does not exist, the processing sequence advances to step S8 whereupon the disconnection of communication is caused due to an abnormal disconnection or time-up and the CPU 81 transmits a reset signal and records, to the memory 82, a message indicating that the current antenna cannot communicate the data (in step S9).

In step S10, the CPU 81 controls the operation for performing the antenna switching processing in which the antenna is returned to the one-previous antenna. In step S11, the operation for connecting the radio communication is tested between the extracorporeal device 5D and the in-body unit 3D.

As a consequence, in step S12, the CPU 81 determines whether or not the operation for connection to the in-body unit 3D is established by radio communication (abbreviated as "in-body unit is found" in Fig. 10). When the connecting operation is established by the radio communication, in step S13, the CPU 81 executes the connecting operation. Then, the CPU 81 returns to step S3 whereupon the antenna for radio communication communicates data with the in-body unit 3D.

When the information (data) on the receiving strength is not obtained, the CPU 81 switches the antenna to an antenna for obtaining the information on the receiving strength so as to assure the communication by the switched antenna, sets the communication state to that of efficiently communicating the data with the in-body unit 3D, and smoothly receives the picked-up image.

When the connecting operation to the in-body unit 3D by the radio communication is not established in step S12, the processing sequence advances to step S14 whereupon the CPU 81 executes the operation for re-connection upon disconnection, and ends the operation for changing the antenna.

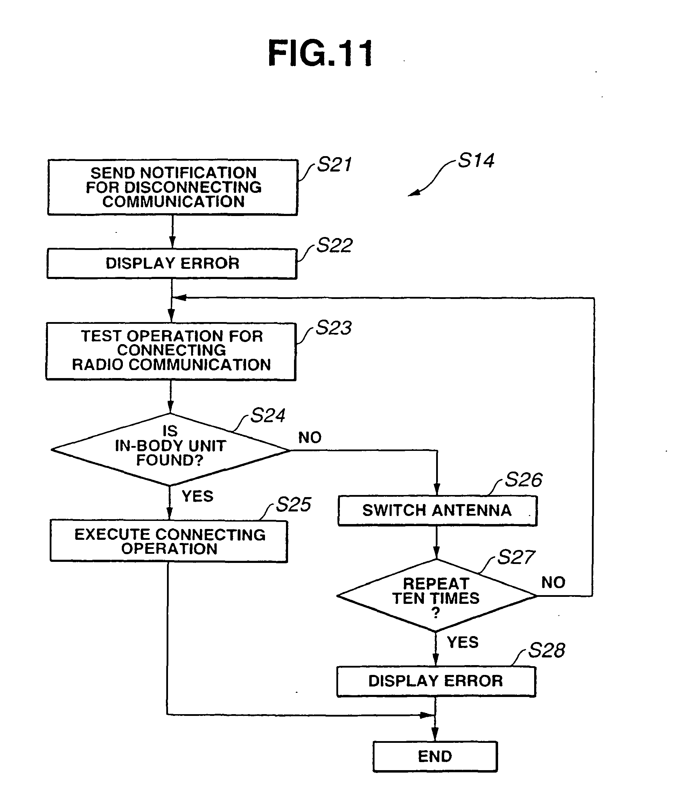

Fig. 11 shows the processing details for the operation of re-connection upon disconnection. In step S21, the in-body unit 3D performs the processing for notifying the communication disconnection to the extracorporeal device 5D. In step S22, (the CPU 81 of) the extracorporeal device 5D displays the disconnection of communication, as an error, on a display panel (not shown) arranged to the recording device 62.

In step S23, (similarly to steps S11 to S13 in Fig. 10), the connection operation by radio communication is tested. As a result, in step S24, it is determined whether or not the connection by the radio communication is established. If the connection is established, in step S25, the connecting operation is implemented and the processing ends.

If the connection is not established, in step S26, the CPU 81 switches the antenna. In step S27, it is determined whether or not the switching operation is repeated ten times, that is, whether or not this switching operation is performed by all the antennas 4a to 4j. If the operation is not performed ten times, the processing routine returns to step S23 whereupon the above-mentioned operation is performed. If the operation is performed ten times and the connection by radio communication is not established, the processing routine shifts to step S28 whereupon the CPU 81 displays the error on the display panel (not shown) in the recording device 62, and ends the processing.

With the above-described operation according to the fifth embodiment, in the processing shown in Fig. 10, the best antenna is selected and the image data picked-up by the in-body unit 3D is received in the best antenna state.

According to the fifth embodiment, the antenna on the extracorporeal device 5D side is switched and the image data is received by the best antenna. The setting for changing the antenna is executed as follows.

### (1) Setting for changing the antenna

In the operation for changing the antenna as described here,
the one to ten antennas 4a to 4j (total number of antennas can be changed) are sequentially attached to the body along the digestive tract.

The object for changing the antenna is premised that the antenna is selected with the preferable communication state, and the detection of position is not included in the object.

A parameter for changing the antenna is set by the image display device 64 and is transferred to the recording device 62 together with patient information.

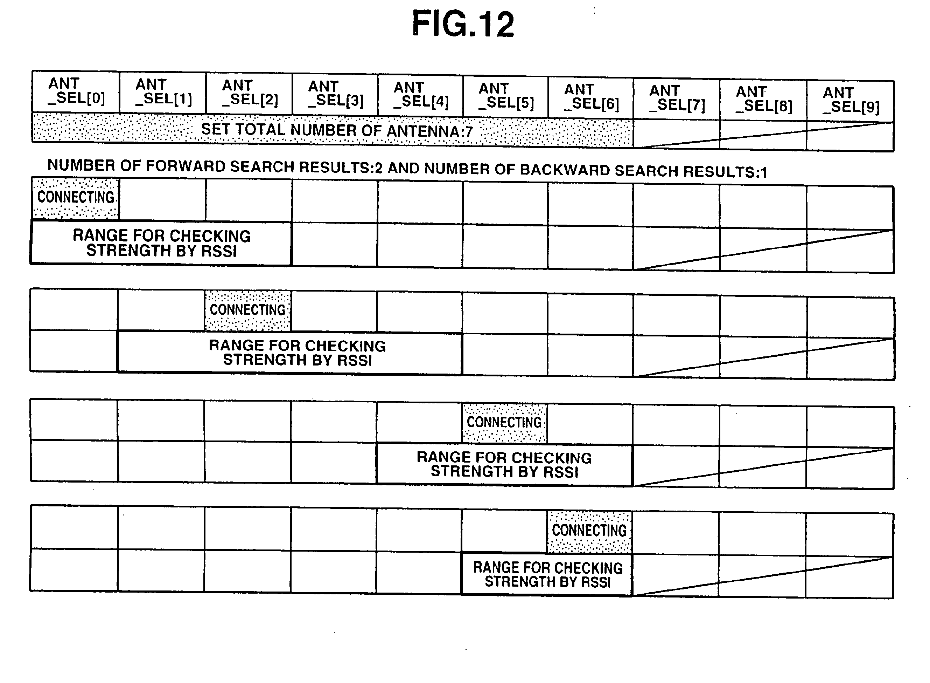

The set items include:
1. Total number of antennas: one to ten
   When the number of antennas is one, referring to Fig. 12, only the antenna selected by ANT_SEL[0] is attached.
2. When the number of antennas is two, the antenna selected by ANT_SELs [0] to [1] is attached.
   Here, the manufacturer performs the operation because the detachment of antenna requires the disassembly and therefore the antenna is not attached to the erroneous position.
3. The number of antennas to be searched in the running direction, namely, in the forward direction of the digestive tract in the processing (hereinafter, abbreviated to RSSI for a brief description) for detecting and obtaining the receiving strength in steps S1 and S2 shown in Fig. 10: zero to nine
4. The number of RSSI to be searched backward: zero to nine
5. In the items 3 and 4, when the number of antennas is zero, the antenna is not changed.
6. The number to be searched backward + the number to be searched forward + one (which is the connected antenna) ≤ total number of antennas, (that is, the number of antennas for RSSI equals to the total number of antennas or less).
7. Determining-reference for selecting an antenna based on the RSSI result
   a. The antenna with the preferable communication state is selected.
   b. When the current antenna and another antenna are determined as preferable ones, the current antenna is continuously used.
   c. When the number of preferable antennas different from the current antenna is two or more, the antenna approximate to the current antenna is selected.
   d. When the number of preferable antennas different from the current antenna is two or more and the back antenna and the front antenna are mixed, the front antenna is preferentially selected.

Next, the operation will be described with example with reference to Fig. 12.

Referring to Fig. 12, the total number of antennas is seven and in the case of changing the antenna, the antennas 4a to 4g are switched by ANT_SEL [0] to ANT_SEL [6], the operation setting for switching the antenna is shown when the number to be searched forward is two and the number to be searched backward is one.

First, the first antenna 4a is connected by ANT_SEL [0]. In this case, the searching backward is not performed, but the two searches forward indicated by RSSI, that is, ANT_SEL [1] and ANT_SEL [2], are performed.

Of course, the operation with RSSI is executed also by ANT_SEL [0] which is connected.

In association with the movement of the in-body unit 3D, the antenna selected to be connected moves. For example, when the antenna 4c of ANT_SEL [2] is connected, the search backward is implemented by ANT_SEL [1] and the search forward is implemented by ANT_SEL [3] and ANT_SEL [4].

Referring to Fig, 12, further, the in-body unit 3D moves and thus the antenna 4f of ANT_SEL [5] is connected, the search backward is implemented by ANT_SEL [4]. When the search forward is prepared by ANT_SEL [6] and the antenna 4g of ANT_SEL [6] is connected, the search backward is performed by ANT_SEL [5].

With the above operation, according to the fifth embodiment, when the in-body unit 3D moves, the best antenna is also effectively selected with the high receiving strength and the image data is received from the in-body unit 3D.

### (Sixth embodiment)

Next, a sixth exemplary embodiment not forming part of the present invention will be described. According to the sixth embodiment, a capsular medical system has the same structure as that of the capsular medical system 1D according to the fifth embodiment shown in Fig. 7. However, the capsular medical system according to the sixth embodiment has the switching operation of the antennas 4a to 4j different to those according to the fifth embodiment shown in Fig. 7.

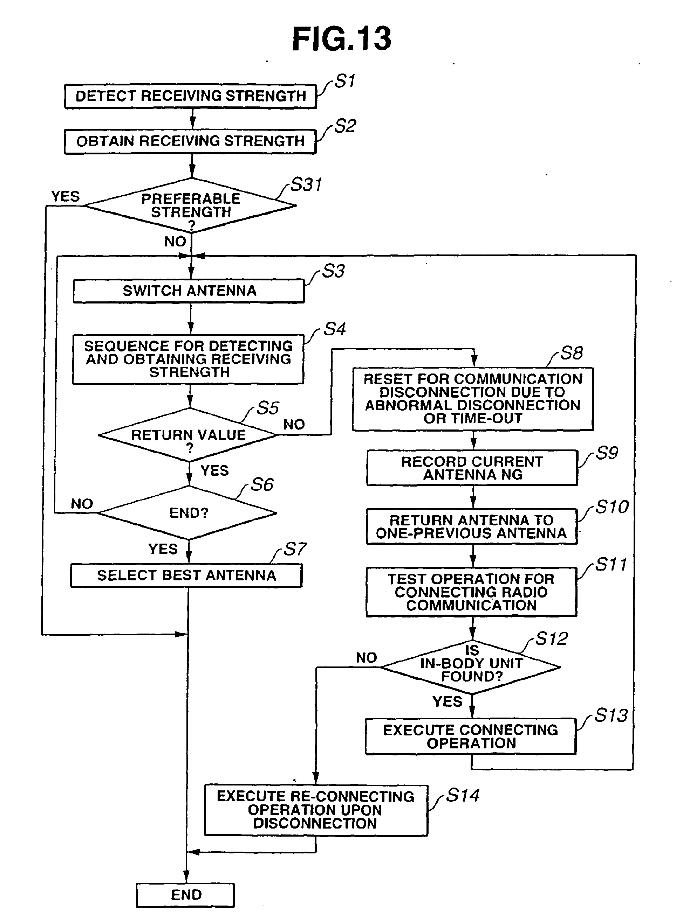

In other words, according to the sixth embodiment, the antenna is switched as shown in Fig. 13.

Similarly to the case shown in Fig. 10, in steps S1 and S2, the receiving strength is detected and obtained. After that, according to the sixth embodiment, in step S31, the CPU 81 in the extracorporeal device 5D determines in the current antenna selecting state (based on the information on the receiving strength in step S2) whether or not the sensitivity is preferable.

When it is determined that the receiving strength is preferable, the operation for switching the antenna ends or the processing sequence returns to step S1. When the antenna with the preferable strength is selected, the state for selecting the antenna maintains.

When it is determined that the strength is not preferable, the processing sequence returns to step S3 whereupon the processing as described above with reference to Fig. 10 is performed.

According to the sixth embodiment, the number of chances for selecting the antenna with unpreferable strength are reduced and the image data is efficiently received from the in-body unit 3D through the antenna with the preferable strength.

The fifth and sixth embodiments use a receiving and transmitting mechanism with the receiving strength suggested by Japanese Unexamined Patent Application Publication No. 2003-135389.

That is, in the state for using the antenna selected by the extracorporeal device 5D, the extracorporeal device 5D sends the signal, the information on the receiving strength (electrical field strength) received and detected by the in-body unit 3D is transmitted (returned) to the extracorporeal device 5D. The extracorporeal device 5D switches the antenna by using the information so as to select the antenna with the highest receiving strength or with preferable receiving state. The image data picked-up by the in-body unit 3D is received by the switched antenna.

The extracorporeal device 5D receives the information on the image data transmitted from the in-body unit 3D and the receiving strength of the antenna selected and used in this case is (detected and) obtained. The antenna is switched based on the obtained information on the receiving strength as described with reference to Fig. 10. The antenna is switched so as to select the antenna with the highest receiving strength or with preferable receiving state approximate thereto. The image data picked-up by the in-body unit 3D may be received by the antenna.

Further, as described according to the first to fourth embodiments, the image data transmitted from the in-body unit is received by the extracorporeal device. The data transfer speed is calculated. The antenna is switched based on the calculated data transfer speed so as to select the antenna with the highest data transfer speed or with preferable data transfer speed approximate thereto. The image data picked-up by the in-body unit may be received.

Furthermore, source coil for generating the magnetic field may be arranged to the antenna portion as the reference of the antennas 4a to 4j shown in Fig. 9. The source coil for generating the magnetic field is used for calculating the shape of an inserting portion of an endoscope. Further, a sense coil may be arranged to the recording device 62 side. The sense coil is used for detecting the magnetic field generated by the source coil and calculating the position of the source coil. And, upon swallowing the in-body unit 3D and initializing operation for examination with the image pick-up operation of the body by the in-body unit 3D, the positions of the antennas 4a to 4j are calculated by the position detection of the source coil. Positional information is recorded to the memory 82 and is used for selecting the antenna upon detecting the communication state (receiving state) or selecting the antenna for detecting the communication state.

That is, when the positional information on each antenna is found, the position of the in-body unit 3D is substantially estimated based on the level of the receiving strength. The positional information is effectively used by the antenna switching or antenna selection. Also, the moving speed of the in-body unit 3D is detected and used for the time interval and timing for detecting the communication state.

According to the first to fourth embodiments, the positional information on the antenna is used and is further used for selecting the antenna for reception.

As mentioned above, the timer 80 or the like can set the time interval for detecting the communication state. Further, the CPU 81 may estimate the current or further proper time interval at which the communication state is detected based on the past data on the antenna switching during moving the in-body unit 3D.

Specifically, when detecting the communication state at a predetermined time interval, at the portion at which the in-body unit 3D moves fast, the timing for properly switching the antenna is missed, that is, the antenna is not switched to the next front antenna but the antenna is possibly switched to further one ahead antenna forward. When the data on the past antenna-switching is stored and the time interval for detection shows a tendency to become shorter, the time interval for next detecting the receiving strength may be set to be short.

In this case, preferably, the precise positional information on the antenna is detected. As mentioned above, the time interval for detecting the future communication state or timing for detecting it may be determined in consideration of the positional information on the antenna and the information on the past antenna-switching.

Further, even in other cases than the case that the means for detecting the positional information on the antenna is arranged, when the clothing 90 is put on, the schematic position of the antenna is determined on the route along the running direction of the digestive tract. Further, the schematic distance between the antennas is determined. Thus, the determined information may be stored in the memory 82 and the time interval for detecting the communication state may be changed and set by referring to the data on the time interval of the antenna switching that is previously detected.

According to the first to sixth embodiments, the image is optically picked up, as the means for obtaining the medical information on the body. However, the present invention is not limited to this and can widely be applied to devices for obtaining medical information on the living body or for performing medical action. Specifically such devices are used for obtaining information on an ultrasonic image with ultrasonic waves, for obtaining pH information by a sensor, and for treatment action by the chemical spray or drug injecting means arranged to the in-body unit through the communication.

### Industrial Applicability

As mentioned above, the capsular medical apparatus according to the present invention monitors the receiving state of the signal which is sent by radio from the capsular in-body unit inserted in the body via the plurality of antennas, thereby preferably receiving the signal which is sent by radio.

## Claims

1. A capsular medical system (1D) comprising:
a capsular in-body unit (3D) having radio communication means which is adapted to be inserted or swallowed to be introduced to the body cavity;
an extracorporeal device (5D) having communication means for performing data communication with the in-body unit (3D), which is adapted to be arranged outside the human body; and
a plurality of antennas (4a to 4j) which are adapted to be arranged near the body surface to communicate with the in-body unit (3D) and which are adapted to be connected to the extracorporeal device (5D),
the extracorporeal device (5D) comprising:
switching means for switching to select one of the plurality of antennas (4a to 4j); and
detecting means for detecting a receiving strength, in the in-body unit (3D), of signals transmitted from the antenna selected by the switching means,
wherein the switching means is adapted to perform the selection of the one of the plurality of antennas (4a to 4j) at an interval of a predetermined time set by a timer (80); and
**characterized in that** the detecting means comprises a RF circuit (94) adapted to transmit signals and receive a signal indicative of a receiving strength of the transmitted signals from the in-body unit (3D) to detect the receiving strength while the one of the plurality of antennas (4a to 4j) is selected by the switching means,
the RF circuit (94) being capable of switching each antenna synchronously at the timing for switching the communication direction of the reception and transmission of each antenna without switching the antenna during the transmission or reception and obstructing the communication.

2. The capsular medical system (1D) according to Claim 1,
wherein the switching means is adapted to select antennas of a number n smaller than a number N of antennas provided at the extracorporeal device (5D) as the plurality of antennas (4a to 4j) from which one antenna is selected.

3. The capsular medical system (1D) according to Claim 2,
wherein the antennas of the number smaller than the number N are determined based on the antenna which currently receive data.

4. The capsular medical system (1D) according to Claim 1,
wherein, when data of the receiving strength are not obtained, the switching means is adapted to select the antenna which has been confirmed to be able to communicate data so as to ensure the data communication.

5. The capsular medical system (1D) according to Claim 1,
wherein the switching means is adapted to switch to select an antenna with the highest receiving strength of the receiving strengths detected by the detecting means.

## Patentansprüche

1. Kapselartiges medizinisches System (1D), aufweisend:
eine kapselartige körperinterne Einheit (3D) mit einem Funkkommunikationsmittel,
das zum Einführen oder Verschlucken eingerichtet ist, damit es in den Körperhohlraum eingebracht werden kann;
ein körperexternes Gerät (5D) mit einem Kommunikationsmittel zum Ausführen des Datenaustauschs mit der körperinternen Einheit (3D), das zum Anordnen außerhalb des menschlichen Körpers eingerichtet ist; und
eine Mehrzahl Antennen (4a bis 4j), die zum Anordnen nahe der Körperoberfläche eingerichtet sind, um mit der körperinternen Einheit (3D) zu kommunizieren, und die zur Verbindung mit dem körperexternen Gerät (5D) eingerichtet sind,
wobei das körperexterne Gerät (5D) aufweist:
ein Schaltmittel zum Schalten, um eine Antenne der Mehrzahl Antennen (4a bis 4j) zu wählen; und
ein Detektormittel zum Erfassen der Empfangsstärke von Signalen, die von den durch das Schaltmittel gewählten Antennen gesendet werden, in der körperinternen Einheit (3D),
wobei das Schaltmittel dazu eingerichtet ist, die Wahl der einen Antenne der Mehrzahl Antennen (4a bis 4j) in einem Intervall einer von einem Zeitgeber (80) eingestellten vorgegebenen Zeit vorzunehmen; und
**dadurch gekennzeichnet, dass** das Detektormittel eine HF-Schaltung (94) aufweist,
die dazu eingerichtet ist, Signale zu senden und ein Signal zu empfangen, das die Empfangsstärke der gesendeten Signale von der körperinternen Einheit (3D) angibt,
um die Empfangsstärke zu erfassen, während der die eine der Mehrzahl Antennen (4a bis 4j) vom Schaltmittel gewählt wird,
wobei die HF-Schaltung (94) jede Antenne synchron mit der Taktung zum Schalten der Kommunikationsrichtung von Empfangen und Senden jeder Antenne ohne Schalten der Antenne während des Sendens oder Empfangens und ohne Behinderung der Kommunikation
schalten kann.

2. Kapselartiges medizinisches System (1D) nach Anspruch 1,
bei dem das Schaltmittel dazu eingerichtet ist, eine Anzahl n Antennen zu wählen, wobei n kleiner ist als die Anzahl N der am körperexternen Gerät (5D) als die Mehrzahl Antennen (4a bis 4j) vorgesehenen Antennen, von denen eine Antenne gewählt wird.

3. Kapselartiges medizinisches System (1D) nach Anspruch 2,
bei dem die Anzahl n < N Antennen auf Basis der Antennen bestimmt wird, die aktuell Daten empfängt.

4. Kapselartiges medizinisches System (1D) nach Anspruch 1,
bei dem dann, wenn keine Daten der Empfangsstärke empfangen werden, das Schaltmittel dazu eingerichtet ist, die Antenne zu wählen, die als kommunikationsfähig bestätigt wurde, um den Datenaustausch sicherzustellen. bei dem das

5. Kapselartiges medizinisches System (1D) nach Anspruch 1,
bei dem das Schaltmittel zum Schalten eingerichtet ist, um eine Antenne mit der höchsten Empfangsstärke der vom Detektormittel erfassten Empfangsstärken zu wählen.

## Revendications

1. Système médical capsulaire (1D) comprenant :
une unité intracorporelle (3D) capsulaire ayant un moyen de communication radio qui est adapté à être inséré ou avalé pour être introduit dans la cavité corporelle ;
un dispositif extracorporel (5D) ayant un moyen de communication pour exécuter une communication de données avec l'unité intracorporelle (3D), qui est adapté à être agencé à l'extérieur du corps humain ; et
une pluralité d'antennes (4a à 4j) qui sont adaptées à être agencées à proximité de la surface du corps pour communiquer avec l'unité intracorporelle (3D) et qui sont adaptées à être connectées au dispositif extracorporel (5D),
le dispositif extracorporel (5D) comprenant :
un moyen de commutation pour commuter afin de sélectionner une de la pluralité d'antennes (4a à 4j) ; et
un moyen de détection pour détecter une force de réception, dans l'unité intracorporelle (3D), de signaux transmis par l'antenne sélectionnée par le moyen de commutation,
dans lequel le moyen de commutation est adapté à effectuer la sélection de l'une de la pluralité d'antennes (4a à 4j) à un intervalle de temps prédéterminé fixé par un temporisateur (80) ; et
**caractérisé en ce que** le moyen de détection comprend un circuit RF (94) adapté à transmettre des signaux et à recevoir un signal indicateur d'une force de réception des signaux transmis de l'unité intracorporelle (3D) afin de détecter la force de réception tandis que l'une de la pluralité d'antennes (4a à 4j) est sélectionnée par le moyen de commutation,
le circuit RF (94) étant apte à commuter chaque antenne de manière synchrone au moment de commutation du sens de communication de la réception et de la transmission de chaque antenne sans commuter l'antenne pendant la transmission ou la réception, ni faire obstacle à la communication.

2. Système médical capsulaire (1D) selon la revendication 1,
dans lequel le moyen de commutation est adapté à sélectionner des antennes d'un nombre n plus petit qu'un nombre N d'antennes prévues au niveau du dispositif extracorporel (5D) comme la pluralité d'antennes (4a à 4j) parmi lesquelles une antenne est sélectionnée.

3. Système médical capsulaire (1D) selon la revendication 2,
dans lequel les antennes du nombre plus petit que le nombre N sont déterminées sur la base de l'antenne qui reçoit actuellement des données.

4. Système médical capsulaire (1D) selon la revendication 1,
dans lequel, lorsque des données de la force de réception ne sont pas obtenues, le moyen de commutation est adapté à sélectionner l'antenne qui a été confirmée être apte à communiquer des données de façon à garantir la communication de données.

5. Système médical capsulaire (1D) selon la revendication 1,
dans lequel le moyen de commutation est adapté à commuter afin de sélectionner une antenne avec la force de réception la plus grande parmi les forces de réception détectées par le moyen de détection.
